# EUROPEAN PATENT SPECIFICATION

(11) **EP 1 660 469 B1**
(45) Date of publication and mention of the grant of the patent: **29.08.2007**
(21) Application number: 04766244.0
(22) Date of filing: 16.07.2004
(51) Int. Cl.: C07D 281/16, C07D 295/20

(54) **PROCESS FOR THE PREPARATION OF 11-(1-PIPERAZINYL)DIBENZO 'B, F! '1 ,4!-THIAZEPINE, AN INTERMEDIATE IN THE SYNTHESIS OF THE ANTIPSYCHOTIC DRUG QUETIAPINE**
VERFAHREN ZUR HERSTELLUNG VON 11-(1-PIPERAZINYL)DIBENZO[B,F][1,4]-THIAZEPIN, EIN ZWISCHENPRODUKT IN DER SYNTHESE DES ANTIPSYCHOTISCHEN ARZNEIMITTELS QUETIAPIN
PROCEDE POUR PREPARER DE LA 11-(1-PIPERAZINYLE)DIBENZO B, F| 1,4| THIAZEPINE, UN INTERMEDIAIRE DANS LA SYNTHESE DU MEDICAMENT ANTIPSYCHOTIQUE QUETIAPINE

(30) Priority: 18.07.2003 PL 36134703
(43) Date of publication of application: 31.05.2006
(73) Proprietor: Helm AG, 20097 Hamburg (DE); INSTYTUT FARMACEUTYCZNY, PL-01-793 Warszawa (PL)
(72) Inventor: KACZMAREK, Lukasz, PL-02-904 Warszawa (PL); BADOWSKA-ROSLONEK, Katarzyna, PL-05-402 Otwock (PL); STOLARCZYK, Elzbieta, PL-59-500 Zlotoryja (PL); SZELEJEWSKI, Wieslaw, PL-03-980 Warszawa (PL)
(74) Representative: UEXKÜLL & STOLBERG
(86) International application number: PCT/EP2004/051520
(87) International publication number: WO 2005/012274

(56) References cited:
- WO-A-01/55125
- US-A- 3 458 516

## Description

This invention relates to a process and intermediate for the preparation of 11-(1-piperazinyl)-dibenzo[b,f] [1,4]thiazepine, which is itself an intermediate in the synthesis of antipsychotic drug Quetiapine.

The known synthesis of Quetiapine (11-{4-[2-(2-hydroxyethoxy)ethyl]-1-piperazinyl}dibenzo[b,f][1,4]thiazepine) described in patents EP 0 240 228 B1 and EP 0 282 236 B1 comprises the cyclization of phenyl 2-(phenylthio)-phenylcarbamate to 10,11-dihydrodibenzo[b,f][1,4]thiazepin-11-one by heating in polyphosphoric acid, followed by transformation of the obtained compound to 11-chloro-derivative by reaction with POCl₃ and the condensation of the chloro-derivative with piperazine. In the last step, the obtained 11-(1-piperazinyl) dibenzo [b,f] [1,4] thiazepine is condensed with 2-(2-chlorethoxy)ethanol to give the final Quetiapine base.

The starting material phenyl 2-(phenylthio)phenylcarbamate is obtained by reaction of 2-aminodiphenyl sulfide with phenyl chloroformate. The synthesis of 2-aminodiphenyl sulfide is described in US 3,050,524 as well as in Helv. Chim. Acta 1965, 48, 336 and comprises the condensation of o-chloronitrobenzene with thiophenol followed by reduction of the nitro group.

The synthesis carried out according to procedures described above requires isolation and extensive purification of intermediates. Starting from o-chloronitrobenzene the total yield of the final product 11-(1-piperazinyl)dibenzo[b,f][1,4]-thiazepine does not exceed 50%. Moreover, 11-chlorodibenzo[b,f][1,4]thiazepine is not very stable and forms by-products, what reduces the yield of the product.

In the International Patent Publication WO 01/55125 the problem of instability is said to be solved by the elimination of 11-chlorodibenzothiazepine from the reaction sequence. The method of preparation comprises the condensation of phenyl 2-(phenylthio)phenylcarbamate with 2-(1-piperazinyl)ethanol, followed by the cyclization of the intermediate and substitution of the hydroxy group with a chlorine atom by reaction with phosphorous oxychloride. The 11-[4-(2-chlorocthyl)-1-piperazinyl]dibenzothiazepine formed is then reacted with ethylene glycol to afford Quetiapine base. A drawback of this method is the use of intermediates which are substituted by hydroxyl groups or chorine atoms which favor undesirable side reactions such as elemination, substitution and dimerisation reactions.

The necessity to isolate intermediates and to repeatedly exchange solvents is disadvantageous both under an ecological and economical point of view and is the main disadvantage of all of the above mentioned methods.

It was surprisingly found that these disadvantages can be overcome and that the production of 11-(1-piperazinyl)dibenzo[b,f][1,4]thiazepine can be simplified by reacting phenyl 2-(phenylthio)phenylcarbamate with piperazine and by effecting cyclisation in a different reaction sequence than in the methods of prior art.

The present invention relates to a process for the preparation of crude 11-(1-piperazinyl)-dibenzo[b,f][1,4]thiazepine which comprises reacting phenyl 2-(phenylthio)phenylcarbamate with piperazine followed by the cyclization of the N-[(2-phenylthio)phenyl]-1-piperazinylcarboxamide formed.

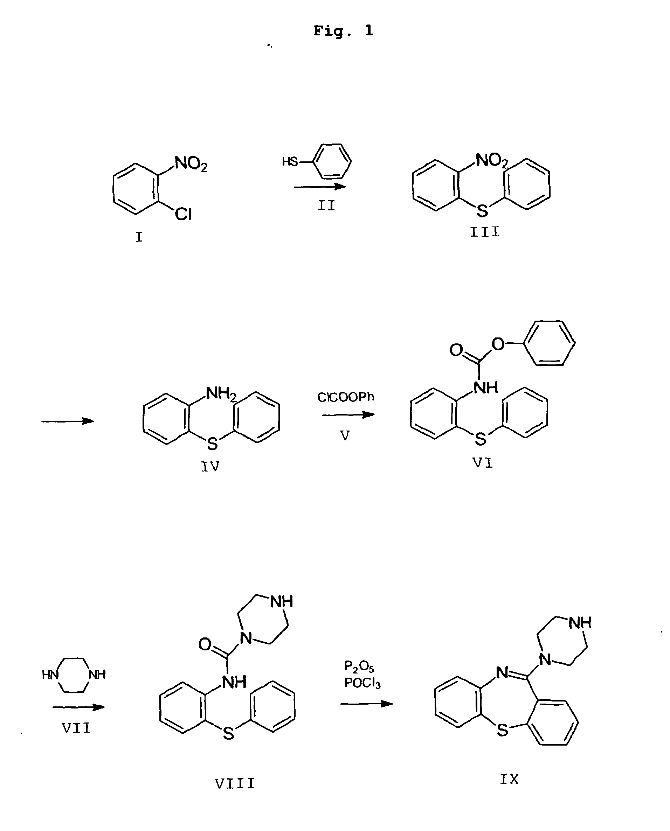

Figure 1 shows a reaction scheme wherein a preferred sequence of reactions for the preparation of 11-(1-piperazinyl)-dibenzo[b,f][1,4]thiazepine IX is shown. Figure 1 also shows a preferred method for the synthesis of phenyl 2-(phenylthio)phenylcarbamate VI which is used as the starting material in the above process.

The 11-(1-piperazinyl)dibenzo[b,f][1,4]thiazepine IX obtained can easily be transformed to Quetiapine or a pharmaceutically acceptable salt thereof by methods known in the art, e.g by reaction with a compound of the formula ZCH₂CH₂OCH₂CH₂OH (X) wherein Z is an atom or group removable as an anion as disclosed in EP 0 240 228 A1. Z advantageously represents a mesyloxy or tosyloxy group, preferably halogen and more preferably a chlorine atom. Thus, the most preferred reagent of formula X is 2-(2-chlorethoxy)ethanol. Quetiapine base can be converted to pharmaceutically acceptable salts by reaction of the base with a pharmaceutically acceptable acid such as hydrochloric acid, maleic acid, fumaric acid, citric acid, phosphoric acid, methane sulphonic acid, and sulphuric acid. A preferred salt is the hemi-fumarate salt.

It was surprisingly found, that 11-(1-piperazinyl)dibenzo-[b,f]1,4]-thiazepine IX prepared according the method of the invention is pure enough to be used for the synthesis of Quetiapine API complying with the high qualitative standards of pharmaceutical products, even if the whole process, starting with the condensation of o-chloronitrobenzene I with thiophenol II to the synthesis of product IX is carried out without purification of intermediates.

The overall process for the preparation of 11-(1-piperazinyl)dibenzo[b,f]1,4]thiazepine IX, which comprises the steps of (a) condensation of o-chloronitrobenzene I with thiophenol II, (b) reducing the nitro group in the sulfide III formed in step (a) to give 2-aminodiphenyl sulfide IV, (c) reacting the resulting 2-aminodiphenyl sulfide IV with phenyl chloroformate V to give phenyl 2-(phenylthio)phenylcarbamate VI, (d) reacting phenyl 2-(phenylthio)phenylcarbamate VI with piperazine VII, followed by (e) the cyclization of the resulting N-[(2-phenylthia)phenyl]-1-piperazinylcarboxamide VIII in the presence of a suitable cyclizing agent to give IX, is also subject of the present invention. The whole process is preferably carried out without the isolation and purification of intermediates. Intermediate N-[(2-phenylthio)phenyl]-1-piperazinylcarboxamide VIII is a further subject of the invention.

The method of the present invention allows the preparation of 11-(1-piperazinyl)dibenzo-[b,f][1,4]thiazepine IX in an overall yield more than 65%, calculated on the basis of o-chloronitrobenzene I as the starting material.

Crude product IX is preferably purified in the following manner: Crude 11-(1-piperazinyl)dibenzo[b,f][1,4]thiazepine is dissolved in aqueous acid, impurities are extracted with an solvent which is not miscible with water, nest the aqueous layer is alkalized and extracted with a similar solvent. The solvent is removed and the oily product can then be transformed into a salt and crystallized. The preferred salt is the hydrochloride.

The preferred solvents which are not miscible with water are chlorinated carbohydrates, in particular dichloromethane.

The condensation of o-chloronitrobenzene with thiophenol in step (a) is preferably carried out in a hydroxyl group(s) containing solvent, e.g. in diluted ethanol, in the presence of a slight excess of an alkaline metal hydroxide (advantageously 1.1 molar equivalent to o-chloronitrobenzene) under reflux.

The reduction of the nitro group of the compound of the formula III is preferably performed without its prior isolation, using an excess of iron dust (advantageously 3.3 molar equivalent to 2-nitrodiphenyl sulfide) in the presence of an acid, preferably hydrochloric acid, advantageously under reflux. Inorganic solid material is then filtered off and the filtrate is acidified, preferably with concentrated hydrochloric acid. The precipitate of hydrochloride is then collected and divided between 10% NaOH and a solvent immiscible with water, e.g. toluene or halogenated hydrocarbon as defined above.

In step (c) the toluene extract is then reacted with phenyl chloroformate, advantageously in the presence of aqueous sodium or potassium carbonate and potassium or sodium hydroxide. The reaction is preferably carried out at a temperature of 5 to 25°C. The organic phase is then separated from the organic phase and the organic phase is washed with water.

Step (d) is preferably accomplished by adding piperazine to the organic layer obtained in step (c), preferably in a proportion of 1.5 to 2 mol of piperazine per 1 mol of phenyl 2-(phenylthio)phenylcarbamate VI, under reflux. The mixture is then washed with aqueous sodium or potassium carbonate, any precipitated by-products are filtered off, and the filtrate is evaporated.

Next the residue is refluxed with cyclizing agent. Suitable cyclizing agents are known in the art. The preferred cyclizing agent is a mixture of phosphorous oxychloride POCl₃ (advantageously 5 to 10 ml per 1 g of the residue) and phosphorous pentoxide P₂O₅ (advantageously 0.5 to lg per lg of the residue). When the reaction is completed, the mixture is diluted with chlorinated hydrocarbon, preferably with dichloromethane, the precipitate is collected and dissolved in water. The acidic solution is extracted with chlorinated hydrocarbon, preferably dichloromethane, phases are separated and the aqueous layer is then alkalised, preferably with sodium hydroxide solution and extracted with the above solvent which is not miscible with water to give 11-(1-piperazinyl)dibenzo[b,f][1,4]thiazepine IX. This product may be further purified by transformation in a salt thereof and crystallization of the salt.

The method of the present invention does not require the use of a complex apparatus and allows the synthesis of IX with a minimal number of operations and only few changes of solvents. This significantly reduces the costs of the overall reaction and is advantageous from an environmental point of view. The process of the present invention preferably comprises only one purification step at the end of the synthesis for purifying 11-(1-piperazinyl)dibenzo[b,f][1,4]thiazepine IX. The product is obtained in a yield of over 65%, calculated on the basis of the starting material o-chloronitrabenzene I.

11-(1-piperazinyl)dibenzo[b,f][1,9]thiazepine IX can then be converted to Quetiapine or a pharmaceutically acceptable salt thereof as described above.

The invention is illustrated by following non-limiting Example:

### Example:

o-Chloronitrobenzene (157 g, 1 mol), technical ethanol (0.25 1) and thiophenol (110g = 102 ml, 1 mol) were placed in a glass reactor (v=21). Then aqueous NaOH (44 g, 1.1 mol in 100 ml of water) was added in two identical portions over a 5 min. time period, with stirring. The temperature increased and reached 60°C after 30 min. The mixture was stirred at ambient temperature for 1 hr and then refluxed for 3 hrs. After cooling to 50°C technical ethanol (0.25 l) and iron dust (185g, 3.3 mol) were added and 36% HCl (20 ml) was dropped into the mixture within 15 min. A high exothermic effect was observed and the mixture boiled for some minutes. After boiling had stopped, the mixture was refluxed for 1 hr and filtered through Celite. The filter cake was extracted with boiling acetone (4 x 150 ml) and discarded. To the combined filtrates 36% HCl (100 ml) was added and the whole mixture was stirred at ambient temperature for 30 min. The precipitate was collected, washed with acetone (3 x 300 ml) and dried on air to give 2-(phenylthio)aniline hydrochloride in yield 210.5 g (89%). This product was powdered and intensively stirred in a mixture of toluene (500 ml) and aqueous NaOH (10%, 500 ml), until the solid was dissolved. The organic layer was separated and the aqueous one extracted once more with toluene (1x500 ml). The combined extracts were washed with water (1 x 500 ml), placed in a glass reactor (V=21) and cooled to 5°C. A solution of phenyl chloroformate (66 ml, 0.53 mol) in toluene (100 ml) was dropped into the reactor at 5-10°C with stirring, during a 2 hrs period. Then another portion of phenyl chloroformate (66 ml, 0.53 mol) in toluene (100 ml) was added dropwise within 1 hr at 5-10°C. Simultaneously, the solution of Na₂CO₃ (82 g) and NaOH (27 g) in water (400 ml) was also dropped into the reaction mixture. When the addition was completed, the mixture was stirred at ambient temperature for 1 hr. When TLC analysis revealed, that all starting amine was consumed, the organic layer was separated and washed consecutively with 10% HCl (2 x 200 ml), water (1 x 200 ml) and brine (1 x 200 ml). To the toluene solution (1 l, containing about 235 g = 0.73 mol of carbamate) was added piperazine (126 g, 1.46 mol) and the mixture was refluxed with stirring for 1 hr (all starting material was consumed). After cooling the mixture was washed with the solution of Na₂CO₃ (150 g) in water (500 ml) and filtered. The filter cake was washed with toluene (200 ml) and the toluene layer was separated from the filtrate. The layer was washed with water (2 x 500 ml) and brine (200 ml) and the solvent was evaporated under reduced pressure. To the residue (about 300 g) POCl₃ (600 ml) and P₂O₅ (300 g) were added. The mixture was refluxed with stirring for 12 hrs, then cooled to 50°C , diluted with dichloromethane (1.5 l) and stirred at ambient temperature for 30 min. The precipitate formed was collected, washed with dichloromethane and dissolved in water with an exothermic effect.

The acidic solution was extracted with dichloromethane (5 x 500 ml), extracts were discarded and the aqueous layer alkalised with 10% NaOH. The alkaline mixture was extracted again with dichloromethane (2 x 1 l and 1 x 0.5 l), extracts were dried over Na₂SO₄ and evaporated to give 11-(1-piperazinyl)dibenzo[b,f][1,4]thiazepine as thick, colourless oil in a yield 198 g (67%).

¹H NMR (CDCl₃. 200 MHz) : 7.54-7.47 (m. 1H) ; 7.40 (d-d, 1H, J=8-2Hz); 7.35-7.29 (m, 4H) ; 7.19 (d-d-d, 1H, J=8-6-1Hz); 7.06 (d-d, 1H, J=8-1Hz); 6.91 (d-d-d, 1H, J=8-6-1Hz); 5.20 (b.s., 1H); 3.84-3.58 (m, 4H); 3.20-2.98 (m, 4H).

MS [-70 eV, EI, m/e(%)]: 295 (5%, M⁺⁻).

## Claims

1. A process for preparation of 11-(1-piperazinyl)dibenzo-[b,f][1,4]thiazepine or a pharmaceutically acceptable salt thereof which comprises reacting phenyl 2-(phenylthio)phenylcarbamate with piperazine and cyclizing the obtained N-[(2-phenylthio)phenyl]-1-piperazinylcarboxamide in a presence of cyclizing agent.

2. The process of claim 1, wherein piperazine is used in an amount of 1.5 to 2 molar equivalents per 1 mol of phenyl 2-(phenylthio)phenylcarbamate.

3. The process of claim 1 or 2, wherein a mixture of phosphor oxychloride and phosphor pentoxide is used as cyclizing agent.

4. The process of any one of the preceding claims wherein the phenyl 2-(phenylthio)phenylcarbamate is obtained by (a) condensation of o-chloronitrobenzene with thiophenol, (b) reducing the nitro group of the reaction product of step (a) to give 2-aminodiphenyl sulfide, (c) reacting the 2-aminodiphenyl sulfide with phenyl chloroformate to form phenyl 2-(phenylthio)phenylcarbamate.

5. The process of claim 4, wherein all steps of the reaction sequence are carried out without the isolation and purification of intermediates.

6. The process of any one of the preceding claims, wherein the crude 11-(1-piperazinyl)-dibenzo[b,f][1,4]thiazepine is dissolved in aqueous acid, the aqueous solution is extracted with a solvent which is not miscible with water, the aqueous layer is alkalised and the product is removed by extraction with a solvent which is not miscible with water, and the solvent is evaporated.

7. The process of claim 6 wherein the obtained 11-(1-piperazinyl)dibenzo[b,f] [1,4]-thiazepine is transformed into a salt thereof and crystallized.

8. The process of claim 6 or 7, wherein the solvent which is not miscible with water is a chlorinated carbohydrate, preferably dichloromethane.

9. The process of claim 7, wherein the purified 11-(1-piperazinyl)dibenzo[b,f][1,4]- thiazepine is transformed into the hydrochloride.

10. The process of any one of the preceding claims wherein the 11-(1-Piperazinyl)dibenzo[b,f][1,4]thiazepine obtained is further reacted with a compound of Lhe formula ZCH₂CH₂OCH₂CH₂OH wherein Z is an atom or group removable as an anion to give 11-{4-[2-(2-hydroxyethoxy)ethyl]-1-piperazinyl}dibenzo[b,f][1,4]thiazepine (Quetiapine).

11. The process of claim 10 wherein Z represents a mesyloxy or tosyloxy group, preferably halogen and more preferably a chlorine atom.

12. The process of claim 10 or 11 wherein the Quetiapine base is converted to a pharmaceutically acceptable salt thereof by reaction of the base with a pharmaceutically acceptable acid selected from hydrochloric acid, maleic acid, fumaric acid, citric acid, phosphoric acid, methane sulphonic acid, and sulphuric acid.

13. N-[(2-Phenylthio)phenyl]-1-piperazinylcarboxamide.

14. Use of N-[(2-phenylthio)phenyl]-1-piperazinylcarboxamide for the preparation of 11-{4-[2-(2-hydroxyethoxy)ethyl]-1-piperazinyl}dibenzo[b,f][1,4]thiazepine.

## Patentansprüche

1. Verfahren zur Herstellung von 11-(1-Piperazinyl)dibenzo[b,f][1,4]thiazepin oder eines pharmazeutisch verträglichen Salzes davon, bei dem man Phenyl-2-(phenylthio)phenylcarbamat mit Piperazin umsetzt und man das erhaltene N-[(2-Phenylthio)phenyl]1-piperazinyl-carboxamid in Anwesenheit eines Cyclisierungsmittels cyclisiert.

2. Verfahren nach Anspruch 1, bei dem man das Piperazin in einer Menge von 1,5 bis 2 Moläquivalenten pro Mol Phenyl-2-(phenylthio)phenylcarbamat verwendet.

3. Verfahren nach Anspruch 1 oder 2, bei dem man eine Mischung von Phosphoroxychlorid und Phosphorpentoxid als Cyclisierungsmittel verwendet.

4. Verfahren nach einem der vorhergehenden Ansprüche, bei dem man das Phenyl-2-(phenylthio)phenylcarbamat dadurch erhält, daß man (a) o-Chlornitrobenzol mit Thiophenol kondensiert, (b) man die Nitrogruppe des Reaktionsprodukts aus Schritt (a) reduziert, um 2-Aminodiphenylsulfid zu ergeben, (c) man das 2-Aminodiphenylsulfid mit Phenylchlorformiat umsetzt, um Phenyl-2-(phenylthio)phenylcarbamat zu bilden.

5. Verfahren nach Anspruch 4, bei dem man alle Schritte der Reaktionssequenz ohne Isolierung und Reinigung von Intermediaten durchführt.

6. Verfahren nach einem der vorangehenden Ansprüche, bei dem man das rohe 11-(1-Piperazinyl)-dibenzo[b,f][1,4]thiazepin in wäßriger Säure löst, man die wäßrige Lösung mit einem nicht wassermischbaren Lösungsmittel extrahiert, man die wäßrige Schicht alkalisiert und das Produkt durch Extraktion mit einem nicht wassermischbaren Lösungsmittel entfernt und man das Lösungsmittel abdampft.

7. Verfahren nach Anspruch 6, bei dem man das erhaltene 11-(1-Piperazinyl)dibenzo[b,f] [1,4]-thiazepin in ein Salz überführt und kristallisiert.

8. Verfahren nach Anspruch 6 oder 7, bei dem das nicht wassermischbare Lösungsmittel ein chlorierter Kohlenwasserstoff, vorzugsweise Dichlormethan ist.

9. Verfahren nach Anspruch 7, bei dem man das gereinigte 11-(1-Piperazinyl)dibenzo[b,f][1,4]thiazepin in das Hydrochlorid überführt.

10. Verfahren nach einem der vorhergehenden Ansprüche, bei dem man das erhaltene 11-(1-Piperazinyl)dibenzo[b,f] [1,4] thiazepin mit einer Verbindung der Formel ZCH₂CH₂OCH₂CH₂OH weiter umsetzt, wobei Z ein Atom oder eine Gruppe ist, die als Anion entfernbar ist, um 11-{4-[2-(2-Hydroxyethoxy)ethyl]-1-piperazinyl}dibenzo[b,f][1,4]thiazepin (Quetiapin) zu bilden.

11. Verfahren nach Anspruch 10, bei dem Z eine Mesyloxy- oder Tosyloxygruppe, vorzugsweise Halogen und besonders bevorzugt ein Chloratom ist.

12. Verfahren nach Anspruch 10 oder 11, bei dem man die Quetiapinbase durch Reaktion der Base mit einer pharmazeutisch verträglichen Säure, die aus Salzsäure, Maleinsäure, Fumarsäure, Zitronensäure, Phosphorsäure, Methansulfonsäure und Schwefelsäure ausgewählt ist, in ein pharmazeutisch verträgliches Salz überführt.

13. N-[(2-Phenylthio)phenyl]-1-piperazinylcarboxamid.

14. Verwendung von N-[(2-Phenylthio)phenyl]-1-piperazinyl-carboxamid zur Herstellung von 11-{4-[2-(2-Hydroxyethoxy)ethyl]-1-piperazinyl}dibenzo[b,f][1,4]thiazepin.

## Revendications

1. Procédé pour la préparation de 11-(1-pipérazinyl)dibenzo[b,fJ[1,4]-thiazépine ou d'un sel pharmaceutiquement acceptable de celle-ci, comprenant la mise en réaction de 2-(phénylthio)phénylcarbamate de phényle avec de la pipérazine et la cyclisation du N-[2-phénylthio)phényl]-1-pipérazinylcarboxamide obtenu, en présence d'un agent de cyclisation.

2. Procédé selon la revendication 1, dans lequel la pipérazine est utilisée en une quantité de 1,5 à 2 équivalents molaires par mole de 2-(phénylthio)phénylcarbamate de phényle.

3. Procédé selon la revendication 1 ou 2, dans lequel on utilise comme agent de cyclisation un mélange d'oxychlorure de phosphore et de pentaoxyde de phosphore.

4. Procédé selon l'une quelconque des revendications précédentes, dans lequel le 2-(phénylthio)phénylcarbamate de phényle est obtenu par (a) condensation de o-chloronitrobenzène avec du thiophénol, (b) réduction du groupe nitro du produit de réaction de l'étape (a) pour l'obtention de sulfure de 2-aminodiphényle, (c) réaction du sulfure de 2-aminodiphényle avec du choloroformiate de phényle, pour l'obtention de 2-(phénylthio)phénylcarbamate de phényle.

5. Procédé selon la revendication 4, dans lequel toutes les étapes de la séquence de réactions sont effectuées sans isolement ni purification des produits intermédiaires.

6. Procédé selon l'une quelconque des revendications précédentes, dans lequel on dissout la 11-(1-pipérazinyl)dibenzo[b,f][1,4]thiazépine brute dans un acide aqueux, on extrait la solution aqueuse avec un solvant non miscible à l'eau, on alcalinise la phase aqueuse et on sépare le produit par extraction avec un solvant non miscible à l'eau, et on évapore le solvant ;

7. Procédé selon la revendication 6, dans lequel la 11-(1-pipérazinyl)-dibenzo[b,f][1,4]thiazépine obtenue est convertie en un de ses sels et cristallisée.

8. Procédé selon la revendication 6 ou 7, dans lequel le solvant non miscible à l'eau est un hydrocarbure chloré, de préférence le dichlorométhane.

9. Procédé selon la revendication 7, dans lequel la 11-(1-pipérazinyl)-dibenzo[b,f][1,4]thiazépine purifiée est convertie en le chlorhydrate.

10. Procédé selon l'une quelconque des revendications précédentes, dans lequel on fait encore réagir la 11-(1-pipérazinyl)dibenzo[b,f][1,4]thiazépine obtenue, avec un composé de formule ZCH₂CH₂OCH₂CH₂OH dans laquelle Z est un atome ou un groupe séparable sous forme d'un anion, pour obtenir la 11-{4-[-2-(2-hydroxyéthoxy)éthyl]-1-pipérazinyl}dibenzo[b,f][1,4]thiazépine(quétiapine).

11. Procédé selon la revendication 10, dans lequel Z représente un groupe mésyloxy ou tosyloxy, de préférence un atome d'halogène et encore mieux un atome de chlore.

12. Procédé selon la revendication 10 ou 11, dans lequel on convertit la quétiapine base en un sel pharmaceutiquement acceptable de celle-ci, en faisant réagir la base avec un acide pharmaceutiquement acceptable choisi parmi l'acide chlorhydrique, l'acide maléique, l'acide fumarique, l'acide citrique, l'acide phosphorique, l'acide méthanesulfonique et l'acide sulfurique.

13. N-[(2-phénylthio)phényl]-1-pipérazinylcarboxamide.

14. Utilisation du N-[2-(phénylthio)phényl]-1-pipérazinylcarboxamide pour la préparation de la 11-{4-[-2-(2-hydroxyéthoxy)éthyl]-1-pipérazinyl}dibenzo[b,f] [1,4]thiazépine.
